# EUROPEAN PATENT SPECIFICATION

(11) **EP 4 433 005 B1**
(45) Date of publication and mention of the grant of the patent: **18.11.2026**
(21) Application number: 22801564.0
(22) Date of filing: 27.10.2022
(51) Int. Cl.: A61F 9/008

(54) **TREATING EYE CONDITIONS WITH SUBTHRESHOLD FEMTOSECOND LASER PULSES**
BEHANDLUNG VON AUGENLEIDEN MIT SUBSCHWELLEN-FEMTOSEKUNDEN-LASERIMPULSEN
TRAITEMENT DE PATHOLOGIES OCULAIRES AVEC DES IMPULSIONS LASER FEMTOSECONDE DE SOUS-SEUIL

(30) Priority: 19.11.2021 US 202163281511 P
(43) Date of publication of application: 25.09.2024
(73) Proprietor: Alcon Inc., 1701 Fribourg (CH)
(72) Inventor: BOR, Zsolt, Lake Forest, California 92630 (US)
(74) Representative: Murgitroyd & Company
(86) International application number: PCT/IB2022/060352
(87) International publication number: WO 2023/089423

(56) References cited:
- US-A1- 2016 235 588
- US-A1- 2017 326 003

## Description

### TECHNICAL FIELD

The present disclosure relates generally to ophthalmic laser surgical systems, and more particularly to treating eye conditions with subthreshold femtosecond laser pulses.

### BACKGROUND

In ophthalmic laser surgery, a surgeon may direct a laser beam into the eye to treat the eye. For example, a laser beam may be directed into the vitreous to treat eye floaters. Eye floaters are clumps of collagen proteins that form in the vitreous. These clumps can disturb vision with moving shadows and distortions. The laser beam may be used to fragment the floaters to improve vision.
Reference is made to the documents US 2017/326003 A1 and US 2016/235588 A1 which have been cited as exemplary of the background state of the art.

### BRIEF SUMMARY

The scope of the invention is in accordance with the appended claims.

In certain embodiments, an ophthalmic laser surgical system for treating a target tissue in an eye includes a target detection system and a laser device. The target tissue has an optical breakdown threshold. The target detection system directs detection beams along a detection beam path towards the target tissue in a vitreous of the eye, and determines a location of the target tissue within the vitreous. The laser device includes a femtosecond laser that generates subthreshold laser pulses that have a pulse energy below the optical breakdown threshold of the tissue. The laser device directs a laser beam comprising the subthreshold laser pulses along a laser beam path towards the target tissue.

Embodiments may include none, one, some, or all of the following features:
The ophthalmic laser surgical system further comprises an xy-scanner. The xy-scanner: receives the detection beams from the target detection system and directs the detection beams along the detection beam path towards an xy-location of a target shadow cast by the target tissue onto the retina of the eye, the xy-location relative to an xy-scanner; and receives the laser beam from the laser device and directs the laser beam along the laser beam path aligned with the detection beam path towards the xy-location of the target shadow.
* The pulse energy is 1 to 100 nanojoules (nJ).
* The subthreshold laser pulses have a duration of 10 to 500 femtoseconds (fs).
* The subthreshold laser pulses have a repetition rate of 1 to 100 megahertz (MHz).
* The laser device configured to direct the laser beam comprising the plurality of subthreshold laser pulses by directing 10 to 100 subthreshold laser pulses towards the same spot of the target tissue. The number N of subthreshold laser pulses at the same target spot may be controlled by a repetition rate f of the laser device, an xy-scanning speed v of a laser spot of the laser beam, and a target spot diameter d according to N = (d*f) /v.
* The laser device comprises a z-focusing component that receives a z-location of the target tissue relative to the retina, and directs a focal point of the laser beam towards the z-location of the target tissue.
* The target detection system comprises an xy-location device that provides an xy-location of a target shadow of the target tissue relative to an xy-scanner, and a z-location device that provides a z-location of the target tissue relative to the retina.
* The xy-location device comprises a scanning laser ophthalmoscopy (SLO) device.
* The z-location device comprises an interferometer device.
* The target tissue comprises a vitreous-retinal traction fiber.
* The target tissue comprises a vitreous floater.
* The laser forms a cloud of low-density free electrons using the subthreshold laser pulses to trigger a chemical reaction that locally disintegrates a portion of the target tissue.
* The laser forms singlet oxygen molecules using the subthreshold laser pulses to cause a chemically reaction within a portion of the target tissue.
* The laser causes a multiphoton chemical reaction using the subthreshold laser pulses to locally disintegrate a portion of the target tissue.
* The laser cause a supersonic thermoelastic wave using the subthreshold laser pulses to locally disintegrate a portion of the target tissue.

In certain embodiments, an ophthalmic laser surgical system for treating a target tissue in an eye comprises a target detection system and a laser device. The target detection system directs detection beams along a detection beam path towards the target tissue in the vitreous of the eye and determines the location of the target tissue. The laser device comprises a femtosecond laser that generates subthreshold laser pulses. The subthreshold laser pulses have a pulse energy below the optical breakdown threshold of the target tissue, e.g., 1 to 100 nanojoules (nJ), a duration of 10 to 500 femtoseconds (fs), and a repetition rate of 1 to 100 megahertz (MHz). The laser device directs a laser beam comprising the subthreshold laser pulses along a laser beam path towards the target tissue by directing 10 to 100 subthreshold laser pulses towards the same target spot of the target tissue.

Embodiments may include none, one, some, or all of the following features:
* The ophthalmic laser surgical system comprises an xy-scanner that: receives the detection beams from the target detection system and directs the detection beams along the detection beam path towards the xy-location of a target shadow cast by the target tissue onto the retina of the eye, the xy-location relative to an xy-scanner; and receives the laser beam from the laser device and directs the laser beam along the laser beam path aligned with the detection beam path towards the xy-location of the target shadow.
* The target detection system comprises: an xy-location device that provides the xy-location of a target shadow of the target tissue, the xy-location related to an xy-scanner; and a z-location device that provides the z-location of the target tissue relative to the retina of the eye.

### BRIEF DESCRIPTION OF THE DRAWINGS

FIGURE 1 illustrates an example of an ophthalmic laser surgical system that may be used to treat an eye, according to certain embodiments;
FIGURE 2 illustrates an example of a retinal image that may be generated by the system of FIGURE 1; and
FIGURE 3 illustrates an example of a method for treating a target tissue in an eye that may be performed by the system of FIGURE 1, according to certain embodiments.

### DESCRIPTION OF EXAMPLE EMBODIMENTS

Referring now to the description and drawings, example embodiments of the disclosed apparatuses, systems, and methods are shown in detail. The description and drawings are not intended to be exhaustive or otherwise limit the claims to the specific embodiments shown in the drawings and disclosed in the description. Although the drawings represent possible embodiments, the drawings are not necessarily to scale and certain features may be simplified, exaggerated, removed, or partially sectioned to better illustrate the embodiments.

Known ophthalmic laser surgical systems include a YAG or femtosecond laser ("femto laser") that generates laser pulses to photodisrupt eye tissue. For example, YAG laser pulses with a 5 millijoule (mJ) pulse energy, 4 nanosecond (ns) pulse duration, and 1.064 micrometer (um) wavelength or femtosecond laser pulses with a 15 to 20 microjoule (uJ) pulse energy and 500 femtosecond (fs) pulse duration can photodisrupt tissue to create cavitation bubbles in the tissue.

However, YAG laser pulses cannot treat floaters closer than 10 millimeters (mm) to the retina without exceeding standard radiation exposure limits. Moreover, Chirped Pulse Amplification with Master Oscillator Power Amplifier (CPA MOPA) femto lasers are needed to produce the pulses that can photodisrupt tissue, and these lasers are prohibitively expensive for certain applications.

Accordingly, the surgical systems described herein include a femtosecond laser that produces subthreshold laser pulses. Subthreshold laser pulses have a pulse energy below the breakdown threshold of the tissue, i.e., the pulse energy at which optical breakdown occurs in the tissue. The pulses do not cause optical breakdown (photodisruption) or form cavitation bubbles. The lower pulse energy allows for destruction of floaters in more situations without overexposing the retina. Moreover, a femtosecond laser is much more affordable and reliable than a CPA MOPA femto laser.

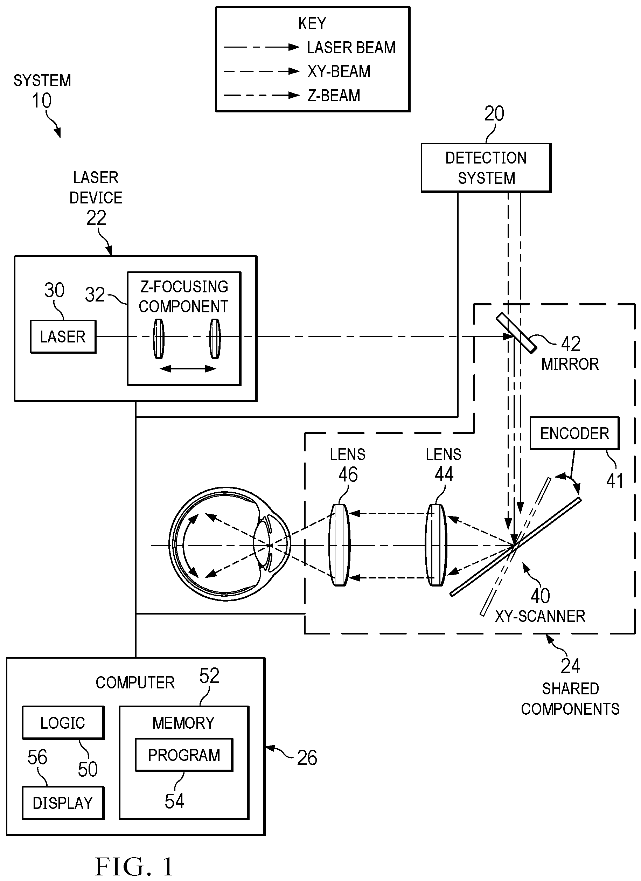

FIGURE 1 illustrates an example of an ophthalmic laser surgical system 10 that may be used to treat an eye, according to certain embodiments. Surgical system 10 may provide any suitable treatment where laser pulses are directed to a target tissue (e.g., vitreous floaters or vitreous-retinal traction fibers) in an eye. Examples of treatments include laser vitreolysis, traction fiber removal, and retinal microsurgery.

As an overview, system 10 includes a target detection system 20, a laser device 22, one or more shared components 24, and a computer 26, coupled as shown. Laser device 22 includes a laser 30 and a z-focusing component 32, coupled as shown. Shared components 24 include an xy-scanner 40, an xy-encoder 41, and optical elements (such as a mirror 42 and lenses 44 and 46), coupled as shown. Computer 26 includes logic 50, a memory 52 (which stores a computer program 54), and a display 56, coupled as shown. In certain embodiments, target detection system 20 and laser device 22 share xy-scanner 40, which allows target detection system 20 and laser device 22 to be co-registered. For ease of explanation, an axis (e.g., optical or visual axis) of the eye approximates a z-axis, which in turn defines enface planes (e.g., xy-planes) substantially orthogonal to the z-axis.

As an overview of operation of system 10, target detection system 20 directs detection beams along a detection beam path towards the target tissue in a vitreous of the eye, and determines the location of the target tissue within the vitreous. Laser device 22 comprises a femtosecond laser that generates subthreshold laser pulses with a pulse energy of 1 to 100 nanojoules (nJ). Laser device 22 directs a laser beam comprising the laser pulses along a laser beam path towards the target tissue.

Turning to the parts of the system, target detection system 20 includes one or more detection devices that detect, locate, and/or image a target tissue and/or a target shadow cast by the target tissue on the retina. To detect, locate, and/or image a target tissue and/or a target shadow, a detection device directs a detection beam along a detection beam path towards the interior of the eye. The interior reflects the detection beam, and the device detects the reflected light and detects, locates, and/or images a target tissue and/or a target shadow. In addition, the detection devices may provide the x, y, and/or z locations of the target tissue and/or a target shadow to another component. For example, an xy-location device provides the xy-location of the target shadow, and a z-location device provides the z-location of the target tissue.

The devices may utilize the same or different technologies (e.g., scanning laser ophthalmoscopy (SLO) and/or interferometry). In certain embodiments, a detection device is an SLO device that provides the xy-location of a target shadow. In certain embodiments, a detection device is an interferometer device with any suitable interferometer, e.g., a Fourier domain type (such as a swept source or a spectral domain type) that utilizes a fast Fourier transform (FFT). Examples of interferometer devices include an optical coherence tomography (OCT) device (such as a swept-source OCT device) and a swept source A-scan interferometer (SSASI) device. A SASSI device performs only A-scans.

Turning to laser device 22, laser 30 includes a femtosecond laser that produces subthreshold laser pulses to perform subthreshold laser surgery (SLS). In contrast, most femtosecond lasers used in ophthalmic surgery cut tissue with above-threshold pulses that cause photodisruption (or plasma mediated ablation). As an example of photodisruption, 3 microjoule (uJ), 500 femtosecond (fs) laser pulses are focused down to about 2 micrometer (um) spots. The peak intensity of the laser pulses at the focus is about 50 TW/cm² (50*10¹² W/cm²), where W represents watt, TW represents terawatt, and cm represents centimeter.

At such extremely high laser intensities, multiphoton absorption can occur. The intensity removes electrons from atoms and molecules and creates a cloud of free electrons. The electric field of the laser pulses accelerate the free electrons by a process called inverse bremsstrahlung, and the accelerated free electrons ionize neutral atoms of the tissue in process called avalanche ionization. As a result of these processes, a high-density, high-temperature (e.g., approximately 5000 K°) plasma is formed at the focus. The high-temperature plasma burns and evaporates a small (e.g., approximately 4 um x 4 um x 50 um ) volume of tissue. The cascade of processes is known as breakdown. Breakdown threshold energy is the minimal laser pulse energy that can cause breakdown.

The high-temperature, high pressure vapors rapidly expand and form a cavitation bubble having a diameter of, e.g., about 200 um. The acceleration of the wall of a cavitation bubble (i.e., the vapor-tissue interface) can achieve an acceleration of 10⁷ m/s², i.e., approximately 1 million times free fall acceleration. The acceleration of bubble walls can also form shock waves in the tissue. The forces of acceleration and the shock waves tear apart a spherical volume of tissue having a diameter of approximately 100 to 200 um, which can be used to disintegrate tissue.

The high-temperature plasma, the rapidly expanding cavitation bubbles, and the shock wave may damage the retina if the focus is near the retina. For this reason, surgery with above-threshold laser pulses is not allowed to be performed very near to or at the surface of the retina. For example, surgery with above-threshold laser pulses is not allowed on the internal limiting membrane, epiretinal membrane, traction fibers connecting the hyaloid membrane and the retina, or retinal drusens.

Accordingly, the femtosecond laser of system 10 produces subthreshold laser pulses for subthreshold laser surgery (SLS), which may be performed closer to the retina as well as farther away. Subthreshold laser pulses have a pulse energy below the breakdown threshold of the tissue, e.g., a pulse energy of 1 to 100 nanojoules (nJ), such as a 50 nJ 500 fs laser pulse energy. The pulses do not cause an optical breakdown, form a high-density, high-temperature plasma, yield a cavitation bubble, or produce shock waves. However, subthreshold laser pulses can yield other effects that can disintegrate tissue. Moreover, several (e.g., greater than 5, e.g., 10) laser pulses may be spatially superimposed on top of each other at the same place at repetition rate of a few MHz to increase disintegration. Subthreshold laser pulses, even when superimposed, disintegrate the tissue locally, only substantially at the focus, so can be used for surgery near the retina.

Subthreshold laser pulses can cause the following effects that disintegrate tissue. Spatial superposition of laser pulses at the same spot can increase effects (1), (2), and (3) that can locally disintegrate tissue at the spot.
(1) Low-density free electrons. Subthreshold laser pulses can form a cloud of low-density free electrons that can trigger a chemical reaction that locally disintegrates tissue.
(2) Singlet oxygen molecules. Subthreshold laser pulses can form singlet oxygen molecules that are extremely chemically reactive.
(3) Multiphoton chemical reaction. Subthreshold laser pulses can cause a multiphoton chemical reaction that disintegrates tissue.
(4) Disruptive local thermoelastic strain. In an example, multiphoton absorption of a laser pulse can increase the temperature of tissue at the focus from, e.g., 37 to 47 centigrade. According to the temperature coefficient of the expansion of water, a focal volume of 2 um diameter may expand by 0.13%, i.e., by 0.0026 um. At a 500 fs rate, the expansion speed may be 0.0026 um/500 fs = 5200 m/s. The speed of sound in the ophthalmic tissue is about 1500 m/s, so the expansion waves are supersonic. Accordingly, subthreshold laser pulses can cause supersonic, explosion-like transient thermoelastic waves that can tear ophthalmic tissue and cause local tissue disintegration of tissue.

Laser 30 generates a laser beam with any suitable wavelength, e.g., in an ultraviolet or infrared range. In certain embodiments, the pulses have a duration of 10 to 500 fs (e.g., 10 to 100, 100 to 200, 200 to 300, 300 to 400, and/or 400 to 500 fs), a pulse energy of 1 to 100 nanojoules (nJ) (e.g., 1 to 5, 5 to 25, 25 to 50, 50 to 75, and/or 75 to 100 nJ), and a repetition rate of 1 to 100 megahertz (MHz) (e.g., 1 to 40, 40 to 60, and/or 60 to 100 MHz). The same spot of the tissue may be exposed to a number N of laser pulses, where N is 10 to 100 (e.g., 10 to 40, 40 to 60, and/or 60 to 100) laser pulses. The number N of pulses at the same target spot may controlled by the repetition rate f of the laser, the xy-scanning speed v of the laser spot of the laser beam, and the target spot diameter d as N = (d*f) /v.

Z-focusing component 32 longitudinally directs the focal point of the laser beam to a specific location in the z-direction. Examples of z-focusing component 32 include a longitudinally adjustable lens, a lens of variable refractive power, an electrically or mechanically tunable lens (e.g., Optotune lens), an electrically or mechanically tunable telescope, or a deformable mirror that can control the z-location of the focal point. Z-focusing component 32 may direct the focal point in any suitable manner. In certain embodiments, z-focusing component 32 receives the z-location of the target tissue from target detection system 20 (and may receive it via computer 26), and directs the laser beam towards the z-location of the target tissue.

Shared components 24 direct detection and laser beams from target detection system 20 and laser device 22, respectively, towards the eye. Because detection and laser beams both use shared components 24, both beams are affected by the same optical distortions (e.g., fan distortion of scanners, barrel or pillow distortions of the scanner lens, refractive distortions from the inner eye surfaces, and other distortions). The distortions affect both beams in the same way, so the distortions are compensated for. This allows for aiming the laser beam using images generated by the detection beam with improved accuracy.

As an example of aiming the laser beam, an image of the eye may include a reticle, which is a graphical overlay (e.g., crosshairs) that indicates where the beam is currently aimed in an enface plane. The user or computer 26 may place the reticle over the target tissue in the image to aim the beam at the target tissue. Target detection system 20 provides the xy-location to xy-scanner 40. Xy-encoder 41 detects the position of xy-scanner 40 to determine the xy-location of the reticle (in encoder units) centered at the target tissue.

As an overview of operation of shared components 24, mirror 42 directs a beam (detection and/or laser beam) towards xy-scanner 40, which transversely directs the beam towards lens 44. Lenses 44 and 46 direct the beam towards eye. Shared components 24 may also provide spectral and polarization coupling and decoupling of detection and laser beams to allow the beams to share the same path.

Turning to the details of shared components 24, xy-scanner 40 transversely directs the focal point of the beam in the x- and y-directions. Xy-scanner 40 changes the angle of incidence of the beam into the pupil, allowing for the beam to cover a wider range within the eye. Xy-scanner 40 may transversely direct the beam in any suitable manner. For example, xy-scanner 40 may include a pair of galvanometrically-actuated scanner mirrors that can be tilted about mutually perpendicular axes. As another example, xy-scanner 40 may include an electro-optical crystal that can electro-optically steer the beam or an acousto-optical crystal that can acousto-optically steer the beam. As another example, xy-scanner 40 may include a fast scanner that can create, e.g., a 3D matrix of laser pulses. Examples of such scanners include a galvo scanner, resonant scanner, or acousto optical scanner. In certain embodiments, xy-scanner 40 receives the xy-location of the target shadow from target detection system 20, and directs the detection and/or laser beam towards the xy-location.

Xy-encoder 41 detects the position of xy-scanner 40 and reports the position as the xy-location. For example, xy-encoder 41 detects the angular orientations of the galvanometer mirrors of xy-scanner 40 in encoder units. Xy-encoder 41 may report the position in encoder units to target detection system 20, laser device 22, and/or computer 26. Since target detection system 20 and laser device 22 share xy-scanner 40, computer 26 can use the encoder units to instruct system 20 and device 22 where to aim their beams, making it unnecessary to perform the computer-intensive conversion from encoder units to a length unit such as millimeters. Xy-encoder 41 may report the positions at any suitable rate, e.g., once every 5 to 50 milliseconds (ms), such as every 10 to 30 or approximately every 20 ms.

Shared components 24 also include optical elements. In general, an optical element can act on (e.g., transmit, reflect, refract, diffract, collimate, condition, shape, focus, modulate, and/or otherwise act on) a laser beam. Examples of optical elements include a lens, prism, mirror, diffractive optical element (DOE), holographic optical element (HOE), and spatial light modulator (SLM). In the example, optical elements include mirror 42 and lenses 44 and 46. Mirror 42 may be a trichroic mirror. Lenses 44 and 46 may be scanning optics of an SLO device.

Computer 26 controls components of system 10 (e.g., target detection system 20, laser device 24, and/or shared components 24) in accordance with a computer program 54. Computer 26 may be separated from components or may be distributed among system 10 in any suitable manner, e.g., within target detection system 20, laser device 24, and/or shared components 24. In certain embodiments, portions of computer 26 that control target detection system 20, laser device 24, and/or shared components 24 may be part of target detection system 20, laser device 24, and/or shared components 24, respectively.

Computer 26 controls the components of system 10 in accordance with a computer program 54. Examples of computer programs 54 include target imaging, target tracking, image processing, target evaluation, retinal exposure calculation, patient education, and insurance authorization programs. For example, computer 26 may use a computer program 54 to instruct target detection system 20, laser device 24, and/or shared components 24 to image a target tissue and focus a laser beam at the target tissue.

In certain embodiments, computer 26 uses an image processing program 54 to perform image processing on an image, e.g., analyze the digital information of the image to extract information from the image. In certain embodiments, image processing program 54 analyzes an image of a target tissue or a target shadow to obtain information about the target tissue. For example, program 54 may detect a target shadow by detecting a darker shape in an image (using, e.g., edge detection or pixel analysis). As another example, program 54 may detect the shape and size of a target shadow, which indicate the size and shape of the target tissue. As another example, program 54 may detect the tone or luminescence of the target shadow, which indicates the density of the target tissue.

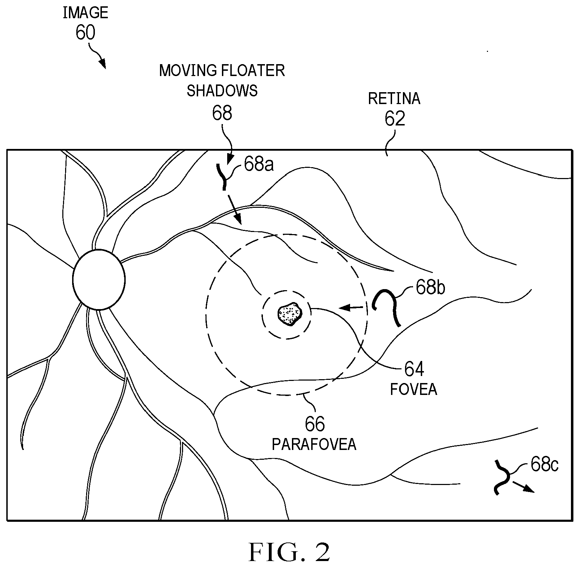

In certain embodiments, computer 26 uses a target evaluation and diagnosis program 54 to evaluate a target tissue, such as a floater, to determine if the target tissue is clinically significant, i.e., affects vision. In certain embodiments, display 56 of computer 26 displays images (such as a video) of a target shadow so a user can evaluate the target tissue. In other embodiments, computer 26 uses image processing to evaluate the target tissue. Target evaluation and diagnosis are as described in more detail with reference to FIGURE 2.

FIGURE 2 illustrates an example of a retinal image 60 that may be generated by system 10 of FIGURE 1. Image 60 shows the retina 62 of an eye, with a foveal region (or fovea) 64 and a parafoveal region (or parafovea) 66. Generally, fovea 64 has a visual angle of approximately +/- one degree, and parafovea 66 has a visual angle of approximately +/- seven degrees. Image 60 also shows floater shadows 68 (68a, 68b, 68c) that floaters cast on retina 62. In general, non-moving shadows are not caused by floaters, and may be caused by, e.g., corneal or lens opacities or anatomical changes of the retina, so floater treatment is not concerned with non-moving shadows.

A floater may be regarded as clinically significant if it can cause a visual disturbance, which can be determined from any suitable features of the floater shadow, e.g., the size and/or density of the shadow, proximity of the shadow to the fovea and/or parafovea, and/or the track of the shadow relative to the fovea and/or parafovea. As an example, a floater can cause a visual disturbance if it permanently or transiently casts a shadow 68 on fovea 64 or can cause distraction or annoyance if it permanently or transiently casts a shadow 68 on parafovea 66. Accordingly, if a floater shadow falls within or is predicted to move within fovea 64 and/or parafovea 66, the floater may be designated as clinically significant. As another example, floater shadow 68 can be used to estimate the size and density of the floater. Larger, denser floaters are more likely to cause a visual disturbance. Thus, a shadow 68 larger than a critical shadow size can indicate a clinically significant floater. A shadow 68 with a higher contrast relative to the background may indicate a clinically significant floater.

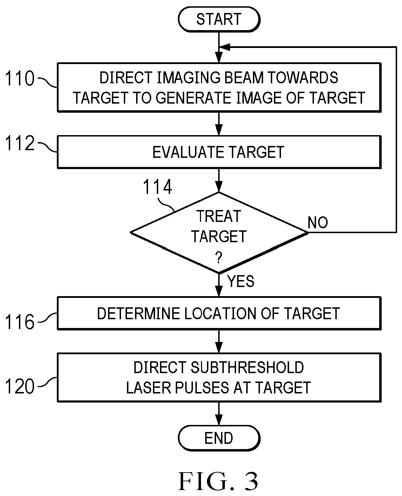

FIGURE 3 illustrates an example of a method for treating a target tissue in an eye that may be performed by system 10 of FIGURE 1, according to certain embodiments. The method starts at step 110, where the target detection system directs a detection beam towards the target tissue shadow to generate an image of the target shadow.

The target tissue is evaluated as to whether it should be treated at step 112. The evaluation may evaluate the target shadow to determine the location, size, shape, and/or density of the target tissue. The target tissue may be treated at step 114. If the target tissue is not to be treated, the method returns to step 110 to continue generating images of the target tissue. If the target tissue is to be treated, the method proceeds to step 116, where the target detection system is used to determine the location of the target shadow.

Subthreshold laser pulses are directed at the target tissue at step 120 to treat (e.g., fragment) the target tissue. Subthreshold laser pulses have a pulse energy below the breakdown threshold of the tissue, so do not cause photodisruption. The pulses treat the target tissue by exposing the same spot to a number N of laser pulses, where N is 10 to 100, to fragment and remove the target tissue.

A component (such as the control computer) of the systems and apparatuses disclosed herein may include an interface, logic, and/or memory, any of which may include computer hardware and/or software. An interface can receive input to the component and/or send output from the component, and is typically used to exchange information between, e.g., software, hardware, peripheral devices, users, and combinations of these. A user interface is a type of interface that a user can utilize to communicate with (e.g., send input to and/or receive output from) a computer. Examples of user interfaces include a display, Graphical User Interface (GUI), touchscreen, keyboard, mouse, gesture sensor, microphone, and speakers.

Logic can perform operations of the component. Logic may include one or more electronic devices that process data, e.g., execute instructions to generate output from input. Examples of such an electronic device include a computer, processor, microprocessor (e.g., a Central Processing Unit (CPU)), and computer chip. Logic may include computer software that encodes instructions capable of being executed by an electronic device to perform operations. Examples of computer software include a computer program, application, and operating system.

A memory can store information and may comprise tangible, computer-readable, and/or computer-executable storage medium. Examples of memory include computer memory (e.g., Random Access Memory (RAM) or Read Only Memory (ROM)), mass storage media (e.g., a hard disk), removable storage media (e.g., a Compact Disk (CD) or Digital Video or Versatile Disk (DVD)), database, network storage (e.g., a server), and/or other computer-readable media. Particular embodiments may be directed to memory encoded with computer software.

Although this disclosure has been described in terms of certain embodiments, modifications (such as changes, substitutions, additions, omissions, and/or other modifications) of the embodiments will be apparent to those skilled in the art. Accordingly, modifications may be made to the embodiments without departing from the scope of the invention. For example, modifications may be made to the systems and apparatuses disclosed herein. The components of the systems and apparatuses may be integrated or separated, or the operations of the systems and apparatuses may be performed by more, fewer, or other components, as apparent to those skilled in the art. As another example, modifications may be made to the methods disclosed herein. The methods may include more, fewer, or other steps, and the steps may be performed in any suitable order, as apparent to those skilled in the art.

To aid the Patent Office and readers in interpreting the claims, Applicants note that they do not intend any of the claims or claim elements to invoke 35 U.S.C. §112(f), unless the words "means for" or "step for" are explicitly used in the particular claim. Use of any other term (e.g., "mechanism," "module," "device," "unit," "component," "element," "member," "apparatus," "machine," "system," "processor," or "controller") within a claim is understood by the applicants to refer to structures known to those skilled in the relevant art and is not intended to invoke 35 U.S.C. §112(f).

## Claims

1. An ophthalmic laser surgical system (10) for treating a target tissue in an eye, comprising:
a target detection system (20) configured to:
direct one or more detection beams along a detection beam path towards the target tissue in a vitreous of the eye, the target tissue having an optical breakdown threshold; and
determine a location of the target tissue within the vitreous; and
a laser device (22) comprising a femtosecond laser (30) configured to generate a plurality of subthreshold laser pulses, the subthreshold laser pulses having a pulse energy below the optical breakdown threshold of the tissue, the laser device (22) configured to:
direct a laser beam comprising the plurality of subthreshold laser pulses along a laser beam path towards the target tissue;
an xy-scanner (40) configured to:
receive the one or more detection beams from the target detection system and direct the one or more detection beams along the detection beam path towards an xy-location of a target shadow cast by the target tissue onto a retina of the eye, the xy-location relative to an xy-scanner (40); and
receive the laser beam from the laser device (22) and direct the laser beam along the laser beam path aligned with the detection beam path towards the xy-location of the target shadow;
wherein the target detection system (20) further comprises:
an xy-location device configured to provide an xy-location of a target shadow of the target tissue, the xy-location related to an xy-scanner (40); and
a z-location device comprising an interferometer device configured to provide a z-location of the target tissue relative to a retina of the eye.

2. The ophthalmic laser surgical system of Claim 1, wherein the pulse energy is 1 to 100 nanojoules.

3. The ophthalmic laser surgical system of Claim 1, the subthreshold laser pulses having a duration of 10 to 500 femtoseconds.

4. The ophthalmic laser surgical system of Claim 1, the subthreshold laser pulses having a repetition rate of 1 to 100 megahertz.

5. The ophthalmic laser surgical system of Claim 1, the laser device (22) configured to direct the laser beam comprising the plurality of subthreshold laser pulses by directing 10 to 100 subthreshold laser pulses towards the same target spot of the target tissue.

6. The ophthalmic laser surgical system of Claim 5, wherein the number N of subthreshold laser pulses at the same target spot is controlled by a repetition rate f of the laser device (22), an xy-scanning speed v of a laser spot of the laser beam, and a target spot diameter d according to N = (d*f) /v.

7. The ophthalmic laser surgical system of Claim 1, the laser device (22) comprising a z-focusing component (32) configured to:
receive a z-location of the target tissue relative to a retina of the eye; and
direct a focal point of the laser beam towards the z-location of the target tissue.

8. The ophthalmic laser surgical system of Claim 1, the xy-location device comprising a scanning laser ophthalmoscopy, SLO, device.

9. The ophthalmic laser surgical system of Claim 1, the target tissue comprising a vitreous-retinal traction fiber.

10. The ophthalmic laser surgical system of Claim 1, the target tissue comprising a vitreous floater.

11. The ophthalmic laser surgical system of Claim 1, the laser (30) configured to:
form a cloud of low-density free electrons using the plurality of subthreshold laser pulses to trigger a chemical reaction that locally disintegrates a portion of the target tissue.

12. The ophthalmic laser surgical system of Claim 1, the laser (30) configured to:
form a plurality of singlet oxygen molecules using the plurality of subthreshold laser pulses to cause a chemically reaction within a portion of the target tissue.

13. The ophthalmic laser surgical system of Claim 1, the laser (30) configured to:
cause a multiphoton chemical reaction using the plurality of subthreshold laser pulses to locally disintegrate a portion of the target tissue.

14. The ophthalmic laser surgical system of Claim 1, the laser (30) configured to:
cause a supersonic thermoelastic wave using the plurality of subthreshold laser pulses to locally disintegrate a portion of the target tissue.

## Patentansprüche

1. Ophthalmisches Laserchirurgiesystem (10) zur Behandlung eines Zielgewebes in einem Auge, das umfasst:
ein Zieldetektionssystem (20), das ausgelegt ist zum:
Richten eines oder mehrerer Detektionsstrahlen entlang eines Detektionsstrahlengangs auf das Zielgewebe in einem Glaskörper des Auges, wobei das Zielgewebe eine optische Abbauschwelle aufweist; und
Bestimmen einer Position des Zielgewebes innerhalb des Glaskörpers; und
eine Laservorrichtung (22), umfassend einen Femtosekundenlaser (30), der dazu ausgelegt ist, eine Vielzahl von Subschwellenlaserpulsen zu erzeugen, wobei die Subschwellenlaserpulse eine Pulsenergie unterhalb der optischen Abbauschwelle des Gewebes aufweisen, wobei die Laservorrichtung (22) ausgelegt ist zum:
Richten eines Laserstrahls, der die Vielzahl von Subschwellenlaserpulsen umfasst, entlang eines Laserstrahlengangs auf das Zielgewebe;
einen xy-Scanner (40), der ausgelegt ist zum:
Empfangen des einen oder der mehreren Detektionsstrahl(en) von dem Zieldetektionssystem und Richten des einen oder der mehreren Detektionsstrahlen entlang des Detektionsstrahlengangs auf eine xy-Position eines Zielschattens, der von dem Zielgewebe auf eine Netzhaut des Auges geworfen wird, wobei die xy-Position auf einen xy-Scanner (40) bezogen ist; und
Empfangen des Laserstrahls von der Laservorrichtung (22) und Richten des Laserstrahls entlang des Laserstrahlengangs, fluchtend mit dem Detektionsstrahlengang, auf die xy-Position des Zielschattens;
wobei das Zieldetektionssystem (20) ferner umfasst:
eine xy-Positionsvorrichtung, die dazu ausgelegt ist, eine xy-Position eines Zielschattens des Zielgewebes bereitzustellen, wobei sich die xy-Position auf einen xy-Scanner (40) bezieht; und
eine z-Positionsvorrichtung, die eine Interferometervorrichtung umfasst, die dazu ausgelegt ist, eine z-Position des Zielgewebes relativ zu einer Netzhaut des Auges bereitzustellen.

2. Ophthalmisches Laserchirurgiesystem nach Anspruch 1, wobei die Pulsenergie 1 bis 100 Nanojoule beträgt.

3. Ophthalmisches Laserchirurgiesystem nach Anspruch 1, wobei die Subschwellenlaserpulse eine Dauer von 10 bis 500 Femtosekunden aufweisen.

4. Ophthalmisches Laserchirurgiesystem nach Anspruch 1, wobei die Subschwellenlaserpulse eine Wiederholungsrate von 1 bis 100 Megahertz aufweisen.

5. Ophthalmisches Laserchirurgiesystem nach Anspruch 1, wobei die Laservorrichtung (22) dazu ausgelegt ist, den Laserstrahl, der die Vielzahl von Subschwellenlaserpulsen umfasst, durch Richten von 10 bis 100 Subschwellenlaserpulsen auf denselben Zielpunkt des Zielgewebes zu richten.

6. Ophthalmisches Laserchirurgiesystem nach Anspruch 5, wobei die Anzahl N von Subschwellenlaserpulsen an demselben Zielpunkt durch eine Wiederholungsrate f der Laservorrichtung (22), eine xy-Abtastgeschwindigkeit v eines Laserpunkts des Laserstrahls und einen Zielpunktdurchmesser d gemäß N = (d*f) /v gesteuert wird.

7. Ophthalmisches Laserchirurgiesystem nach Anspruch 1, wobei die Laservorrichtung (22) eine z-fokussierende Komponente (32) umfasst, die zu Folgendem ausgebildet ist:
Empfangen einer z-Position des Zielgewebes relativ zur Netzhaut des Auges; und
Richten eines Brennpunkts des Laserstrahls auf die z-Position des Zielgewebes.

8. Ophthalmisches Laserchirurgiesystem nach Anspruch 1, wobei die xy-Positionsvorrichtung eine Scanning-Laser-Ophthalmoskopie (SLO)-Vorrichtung umfasst.

9. Ophthalmisches Laserchirurgiesystem nach Anspruch 1, wobei das Zielgewebe eine Glaskörper-Retina-Traktionsfaser umfasst.

10. Ophthalmisches Laserchirurgiesystem nach Anspruch 1, wobei das Zielgewebe eine Glaskörpertrübung umfasst.

11. Ophthalmisches Laserchirurgiesystem nach Anspruch 1, wobei der Laser (30) für Folgendes ausgebildet ist:
Bilden einer Wolke aus freien Elektronen niedriger Dichte unter Verwendung der Vielzahl von Subschwellenlaserpulsen, um eine chemische Reaktion auszulösen, die einen Teil des Zielgewebes lokal abbaut.

12. Ophthalmisches Laserchirurgiesystem nach Anspruch 1, wobei der Laser (30) für Folgendes ausgebildet ist:
Bilden einer Vielzahl von Singulett-Sauerstoffmolekülen unter Verwendung der Vielzahl von Subschwellenlaserpulsen, um eine chemische Reaktion innerhalb eines Teils des Zielgewebes zu bewirken.

13. Ophthalmisches Laserchirurgiesystem nach Anspruch 1, wobei der Laser (30) für Folgendes ausgebildet ist:
Bewirken, dass eine chemische Multiphotonenreaktion unter Verwendung der Vielzahl von Subschwellenlaserpulsen einen Teil des Zielgewebes lokal abbaut.

14. Ophthalmisches Laserchirurgiesystem nach Anspruch 1, wobei der Laser (30) für Folgendes ausgebildet ist:
Bewirken, dass eine thermoelastische Überschallwelle unter Verwendung der Vielzahl von Subschwellenlaserpulsen einen Teil des Zielgewebes lokal abbaut.

## Revendications

1. Système chirurgical ophtalmique à laser (10) destiné au traitement d'un tissu cible dans un œil, comprenant :
un système de détection de cible (20) configuré pour :
diriger un ou plusieurs faisceaux de détection le long d'un trajet de faisceau de détection vers le tissu cible dans un vitré de l'œil, le tissu cible ayant un seuil de claquage optique ; et
déterminer un emplacement du tissu cible à l'intérieur du vitré ; et
un dispositif laser (22) comprenant un laser femtoseconde (30) configuré pour générer une pluralité d'impulsions laser inférieures au seuil, les impulsions laser inférieures au seuil ayant une énergie d'impulsion inférieure au seuil de claquage optique du tissu, le dispositif laser (22) étant configuré pour :
diriger un faisceau laser comprenant la pluralité d'impulsions laser inférieures au seuil le long d'un trajet de faisceau laser vers le tissu cible ;
un scanner xy (40) configuré pour :
recevoir le ou les faisceaux de détection en provenance du système de détection de cible et diriger le ou les faisceaux de détection le long du trajet du faisceau de détection vers un emplacement xy d'une ombre cible projetée par le tissu cible sur une rétine de l'œil, l'emplacement xy étant relatif à un scanner xy (40) ; et
recevoir le faisceau laser en provenance du dispositif laser (22) et diriger le faisceau laser le long du trajet de faisceau laser aligné sur le trajet de faisceau de détection vers l'emplacement xy de l'ombre cible ;
dans lequel le système de détection de cible (20) comprend en outre :
un dispositif de localisation xy configuré pour fournir un emplacement xy d'une ombre cible du tissu cible, l'emplacement xy étant associé à un scanner xy (40) ; et
un dispositif de localisation z comprenant un dispositif interférométrique configuré pour fournir un emplacement z du tissu cible par rapport à une rétine de l'œil.

2. Système chirurgical ophtalmique à laser selon la revendication 1, dans lequel l'énergie d'impulsion est de 1 à 100 nanojoules.

3. Système chirurgical ophtalmique à laser selon la revendication 1, les impulsions laser inférieures au seuil ayant une durée de 10 à 500 femtosecondes.

4. Système chirurgical ophtalmique à laser selon la revendication 1, les impulsions laser inférieures au seuil ayant une fréquence de répétition de 1 à 100 mégahertz.

5. Système chirurgical ophtalmique à laser selon la revendication 1, le dispositif laser (22) étant configuré pour diriger le faisceau laser comprenant la pluralité d'impulsions laser inférieures au seuil en dirigeant 10 à 100 impulsions laser inférieures au seuil vers le même point cible du tissu cible.

6. Système chirurgical ophtalmique à laser selon la revendication 5, dans lequel le nombre N d'impulsions laser inférieures au seuil au niveau du même point cible est commandé par une fréquence f de répétition du dispositif laser (22), une vitesse v de balayage xy d'un point laser du faisceau laser, et un diamètre d de point cible selon N = (d*f)/v.

7. Système chirurgical ophtalmique à laser selon la revendication 1, le dispositif laser (22) comprenant un composant de focalisation en z (32) configuré pour :
recevoir un emplacement z du tissu cible par rapport à une rétine de l'œil ; et
diriger un point focal du faisceau laser vers l'emplacement z du tissu cible.

8. Système chirurgical ophtalmique à laser selon la revendication 1, le dispositif de localisation xy comprenant un dispositif d'ophtalmoscopie laser à balayage, SLO.

9. Système chirurgical ophtalmique à laser selon la revendication 1, le tissu cible comprenant une fibre de traction vitréo-rétinienne.

10. Système chirurgical ophtalmique à laser selon la revendication 1, le tissu cible comprenant un corps flottant du vitré.

11. Système chirurgical ophtalmique à laser selon la revendication 1, le laser (30) étant configuré pour :
former un nuage d'électrons libres de faible densité à l'aide de la pluralité d'impulsions laser inférieures au seuil pour déclencher une réaction chimique qui désintègre localement une partie du tissu cible.

12. Système chirurgical ophtalmique à laser selon la revendication 1, le laser (30) étant configuré pour :
former une pluralité de molécules d'oxygène singulet à l'aide de la pluralité d'impulsions laser inférieures au seuil pour provoquer une réaction chimique dans une partie du tissu cible.

13. Système chirurgical ophtalmique à laser selon la revendication 1, le laser (30) étant configuré pour :
provoquer une réaction chimique multiphotonique à l'aide de la pluralité d'impulsions laser inférieures au seuil pour désintégrer localement une partie du tissu cible.

14. Système chirurgical ophtalmique à laser selon la revendication 1, le laser (30) étant configuré pour :
provoquer une onde thermoélastique supersonique à l'aide de la pluralité d'impulsions laser inférieures au seuil pour désintégrer localement une partie du tissu cible.
